# FASCICULE DE BREVET EUROPEEN

(11) **EP 3 237 012 B1**
(45) Date de publication et mention de la délivrance du brevet: **15.07.2020**
(21) Numéro de dépôt: 15825854.1
(22) Date de dépôt: 18.12.2015
(51) Int. Cl.: A61K 47/10, A61K 9/08, A61K 31/20, A61K 31/201, A61K 31/202, A61K 31/366, A61K 36/53, A61P 25/22

(54) **COMPOSITION APAISANTE POUR ANIMAUX COMPRENANT AU MOINS UN ACIDE GRAS ET DE LA NEPETALACTONE**
BESÄNFTIGENDE ZUSAMMENSETZUNG FÜR TIERE MIT MINDESTENS EINER FETTSÄURE UND NEPETALACTON
SOOTHING COMPOSITION FOR ANIMALS, COMPRISING AT LEAST ONE FATTY ACID AND NEPETALACTONE

(30) Priorité: 24.12.2014 FR 1463323
(43) Date de publication de la demande: 01.11.2017
(73) Titulaire: VIRBAC, 06516 Carros (FR)
(72) Inventeur: MONGINOUX, Patricia, 06270 Villeneuve Loubet (FR); GUERRET, Olivier, 46170 Pern (FR); DUFOUR, Samuel, 64300 Orthez (FR)
(74) Mandataire: Gevers & Orès
(86) Numéro de dépôt international: PCT/IB2015/059793
(87) Numéro de publication internationale: WO 2016/103133

(56) Documents cités:
- EP-A1- 0 724 832
- EP-A1- 1 066 829
- AU-A4- 2007 100 281
- GB-A- 2 345 635
- US-A1- 2011 150 822
- "Feliway Solution à Vaporiser 60ml", Chezpara.fr , 2 juillet 2014 (2014-07-02), XP002742634, Extrait de l'Internet: URL:http://web.archive.org/web/20140702025 124/http://chezpara.fr/Discount/Divers/Vet erinaire/Comportement/Feliway-Solution-a-V aporiser-60ml.html [extrait le 2015-07-21]
- PAGEAT PATRICK ET AL: "Current research in canine and feline pheromones", VETERINARY CLINICS OF NORTH AMERICA: SMALL ANIMAL PRACTICE, SAUNDERS, PHILADELPHIA, US, vol. 33, no. 2, 1 mars 2003 (2003-03-01), pages 187-211, XP008173788, ISSN: 0195-5616, DOI: 10.1016/S0195-5616(02)00128-6 [extrait le 2003-03-12]
- TUCKER A O ET AL: "Catnip and the catnip response", ECONOMIC BOTANY, NEW YORK BOTANICAL GARDEN, BRONX, NY, US, vol. 42, 1 janvier 1988 (1988-01-01), pages 214-231, XP008050438, ISSN: 0013-0001

## Description

La présente invention concerne de nouvelles compositions apaisantes pour animaux, en particulier les chats, elle vise plus particulièrement des compositions associant des acides gras constitutifs des phéromones faciales synthétiques de chat à de la népétalactone. Ces compositions ont la particularité de présenter des propriétés apaisantes pour l'animal dans le cas de stress et sont stables pendant toute la durée de leur utilisation.

La problématique d'apaisement des animaux, qu'ils soient de compagnie ou de rente, est une problématique importante qui a été largement étudiée dans l'Art Antérieur. En effet, les produits utilisés représentent un poids économique important et il existe pour les propriétaires un besoin de combattre les comportements et les symptômes désagréables des animaux domestiques, en particulier les chiens et/ou les chats et, pour les éleveurs un intérêt économique à apaiser les animaux de production en vue d'augmenter leur productivité.

Par exemple, les félins en général et les chats en particulier présentent des symptômes de stress ou d'anxiété très spécifiques et instinctifs, tels que les griffures ou griffades, ou encore les marquages urinaires. Ces derniers consistent à uriner dans différents espaces des pièces dans le but, notamment, de marquer son territoire et de créer ainsi un espace plus rassurant pour l'animal. Par ces actions, les chats déposent des molécules odoriférantes qu'ils peuvent identifier. Cependant, il est évident que ces différents comportements représentent des problèmes de santé et de salubrité importants pouvant incommoder les propriétaires des chats.

Divers travaux ont permis d'identifier les molécules que les chats utilisent pour marquer leur territoire afin de mettre au point des compositions permettant de les apaiser, et de limiter les comportements incommodants pour l'Homme.

Ainsi les premiers travaux de P. Pageat ont mis en évidence que le chat dépose des phéromones faciales, dont la fraction F3, qui jouent un rôle apaisant sur le chat. Le développement d'une formule contenant une reconstitution synthétique de cette fraction F3 en un mélange d'acide pimélique, d'acide azélaique, d'acide palmitique et d'acide oléique a ensuite été décrit dans le document EP0724832. Dans ce document, la fraction F3 est associée à des extraits de valériane dans des mélanges en émulsion. La valériane ayant un caractère attractif pour les chats, elle attire le chat vers le lieu de diffusion où la fraction F3 joue son rôle d'apaisant.

Des travaux sur la méthode de diffusion des compositions à base de la reconstitution synthétique de la fraction F3 ont ensuite été conduits afin de résoudre des problèmes inhérents à ces molécules et à leur mode d'application. Ces problèmes sont variés : stabilité chimique (estérification ou oxydation des acides gras), stabilité physique (cristallisation), vitesse de diffusion (du fait de la grande viscosité de la fraction F3 pure).

Ainsi, dans le document EP1066829, les auteurs expliquent que des compositions comprenant des acides gras tels que ceux de la fraction F3 sont très visqueuses et nécessitent une solubilisation dans un alcool. La stabilité chimique des acides gras en présence d'alcool étant problématique, les auteurs ont choisi de formuler la fraction F3 dans des microémulsions à l'aide de tensio-actifs de type stéarate.

Une autre approche, décrite dans le document WO2009144321, a consisté à déposer la fraction F3 sur des supports cellulosiques qui sont ensuite utilisés dans des diffuseurs électriques pour réguler thermiquement un profil de relargage de la fraction F3.

A part ces travaux pionniers, d'autres substances sémio chimiques ont été découvertes. Par exemple, dans le document WO9937297, les auteurs rapportent que des substances issues des glandes mammaires de mammifères ont elles aussi un effet apaisant. Les compositions chimiques identifiées sont encore une fois des mélanges d'acides gras, notamment des mélanges d'acides linoléique, oléique et palmitique. Les travaux illustrés dans les documents US20110150822 et US20130210927 sont d'autres exemples de ce genre de compositions, où cette fois ci sont divulgués des mélanges d'acides gras et de squalène ou encore une interomone.

Ces compositions posent toutefois les mêmes problématiques de formulation et de relargage contrôlé pendant toute la durée de l'exposition au traitement apaisant. Par ailleurs, la communication olfactive chez les chats ne peut être réduite aux seules phéromones, les odeurs classiques influençant aussi le comportement des félins. Néanmoins, depuis que l'attention s'est portée sur la communication phéromonale, la communication olfactive classique a été mise de côté par les sciences vétérinaires.

La népétalactone est un attractant-apaisant connu de l'homme du métier. La réponse à la népétalactone est restreinte à une stimulation olfactive seulement chez les chats, cependant sans impliquer l'organe voméronasal (Hart and Leedy 1985). Par ailleurs, cette réponse est indépendante de l'état des gonades et est clairement associée au comportement de plaisir chez le chat (Hatch 1972, Hart 1974).

L'odeur de la népétalactone est hautement attractive pour différents félins (que ce soit chez les chats sauvages ou domestiques) de manière dose dépendante, même à de faibles dosages chez les chats domestiques (0,01-0,1 mg) et l'intérêt diminue aux environs de la dose 0,001 mg. Il est connu qu'une exposition à la népétalactone améliore le bien-être général des chats, facilite le comportement enjoué et les interactions sociales, et induit des effets apaisants. D'autre part, la népétalactone est un attractant connu. Cette molécule est le principe actif qui explique l'attractivité des plantes herbacées de la famille *Nepeta.* La népétalactone est un terpénoide possédant 3 centres asymétriques, le ratio des différents isomères variant selon les variétés de *Nepeta* dont elle est issue. La nomenclature de ces isomères est définie par la numérotation des atomes de la népétalactone :

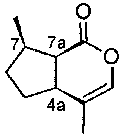

Il existe huit stéréoisomères de la népétalactone, mais les produits issus de la nature présentent une configuration S sur le carbone 7. Il s'agit donc des isomères 4aβ,7α,7aβ; 4aβ,7α,7aβ; 4aα,7α,7aα; 4aα,7α,7aβ (aussi dénommés respectivement cis,cis; trans,trans; cis,trans ; trans,cis). On trouve dans l'article de F. Senatore et al. (Chemistry & Biodiversity, vol 8, 2011, p. 1987) une revue complète des sources naturelles de népétalactone (et donc des différents isomères présents dans chaque espèce de *Nepeta*).

| | | | |
|---|---|---|---|
| 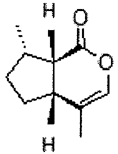 | 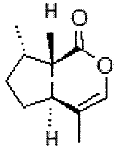 | 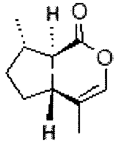 | 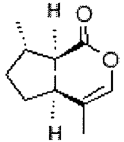 |
| Cis,Cis | Trans,Cis | Trans, Trans | Cis, Trans |
| 4aβ,7α,7aβ | 4aα,7α,7aβ | 4aβ,7α,7aβ | 4aα,7α,7aα |

La *Nepeta cataria,* connue comme étant l'herbe à chat commune, présente la plus grande concentration en népétalactone. Elle peut contenir, selon son origine, les 4 isomères 4aα,7α,7aα ; 4aβ,7α,7aβ ; 4aα,7α,7aβ ; 4aβ,7α,7aα mais aussi l'isomère 4aβ,7β,7aα. L'abondance des isomères dans l'herbe à chat peut dépendre de la région de culture, mais il semble que tous les isomères de la népétalactone aient un effet attractant sur le chat.

De manière industrielle, la népétalactone peut être obtenue soit de manière synthétique soit par extraction de l'herbe à chat comme résumé dans la revue Birkett et al. (Phytochemistry 62(2003) 651-656).

Une composition homogène de népétalactone associée à des acides gras, de préférence à l'analogue synthétique de la fraction F3, et permettant un relargage homogène par diffusion représente une solution idéale pour prévenir et apaiser le stress chez le chat et pour faire disparaitre les symptômes qui y sont associés avec une grande efficacité.

En effet, non seulement la népétalactone, de par sa nature attractive pour le chat, va permettre d'attirer ce dernier dans le lieu où la composition a été diffusée et les acides gras, de préférence l'analogue synthétique de la fraction F3, vont permettre de prévenir et d'apaiser les chats vis-à-vis du stress ressenti, ce qui permettra entre autre d'éviter les futurs marquages, tels que les marquages urinaires et les griffades. Mais en outre, comme cela a été découvert, la népétalactone, sous la forme purifiée d'origine naturelle ou d'origine synthétique ou encore sous la forme d'extraits de plantes, en particulier de la famille *Nepeta,* permet de renforcer significativement l'effet apaisant des acides gras et concoure à apaiser le stress chez le chat pour faire disparaitre les symptômes qui y sont associés avec une efficacité améliorée.

La difficulté pour mettre au point de telles compositions vient du fait que la népétalactone appartient à la famille chimique des lactones. Le schéma suivant explique la réactivité de ces molécules :

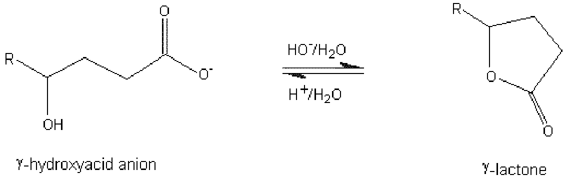

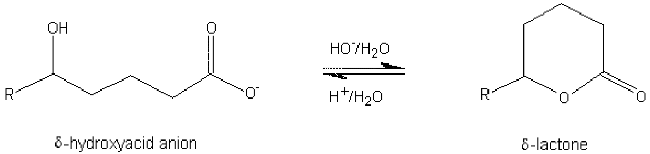

Les lactones se forment en milieu basique et s'hydrolysent en milieu acide. C'est aussi le cas des lactones de la famille de la népétalactone (voir A. Bianco Pure & Appl. Chem., Vol. 66, 1011 1, pp. 2335-2338, 1994). En présence d'acide carboxylique, il est encore possible de subir des transestérifications. Un moyen d'éviter ces transestérifications pourrait consister à utiliser des sels d'acides basiques plutôt que leur forme acide. Cependant, les sels d'acides ne peuvent être utilisés pour une phéromone apaisante sans poser des problèmes de distribution du produit puisqu'ils ne s'évaporent pas. En effet, pour que la népétalactone puisse efficacement jouer un rôle d'attraction de l'animal, il est nécessaire qu'elle diffuse dans l'atmosphère afin d'être sentie et détectée par l'animal pendant toute la durée nécessaire de l'exposition à la composition apaisante. Aussi, les compositions selon l'invention sont des solutions qui permettent une évaporation satisfaisante des différents constituants de la composition, à la fois les acides gras et la népétalactone.

Par ailleurs, la durée d'exposition ou de traitement des chats, pour les habituer à un nouvel environnement, est de l'ordre d'un mois, ce qui constitue une très longue durée dans le référentiel des réactions chimiques entre les différents constituants d'une composition complexe. Il s'avère donc être très difficile de combiner la népétalactone avec une reconstitution synthétique de la fraction F3 de la phéromone faciale du chat, voire même avec un ou plusieurs acides gras dans une composition liquide homogène dont les constituants ne peuvent être distingués à l'œil nu après agitation et efficace pendant la durée requise sans qu'il n'y ait de transformation chimique majeure pendant la durée du traitement. De telles transformations ont pour effet de dénaturer les constituants et particulièrement la népétalactone qui devient alors indisponible. La stabilité physique et chimique de la népétalactone au sein de la composition apaisante doit donc être assurée de manière satisfaisante.

Dans la demande GB2345635 les auteurs ont tenté de résoudre le problème en encapsulant une association comprenant notamment de la népétalactone et des acides gras. Cependant une telle composition n'est pas facile à mettre en œuvre car l'encapsulation est un procédé complexe et coûteux. De plus, l'encapsulation prévient la diffusion des acides gras, ce qui limite le mode d'action du système apaisant à un mode de contact, la friction du chat sur les capsules étant dans ce cas nécessaire pour relarguer les acides gras.

Le document AU2007100281 décrit une composition calmante pour les animaux, capable de réduire le dégagement de mauvaises odeurs résultant de l'activité microbienne de microorganismes présents sur l'animal, d'absorber les éventuelles mauvaises odeurs de l'animal et de le parfumer ; il s'agit d'une composition aqueuse complexe associant des phéromones animales avec une substance attractive telle qu'un extrait de *Nepeta cataria* et comprenant en particulier des cyclodextrines non complexées capables de capter des substances malodorantes, des antibactériens incluant la chlorhexidine et des antimicrobiens afin d'éliminer les microorganismes et du parfum. La stabilité de cette composition, et en particulier celle de l'extrait de *Nepeta cataria,* n'est toutefois pas décrite, ni sa durée d'action. En outre, la proportion des différents ingrédients contenus dans cette composition est très différente de celle des compositions selon l'invention.

A la connaissance de la Demanderesse, il n'existe pas à ce jour de composition liquide homogène de népétalactone et d'acide gras permettant un relargage par diffusion ; elle s'est donc donné pour objectif de remédier à ce manque.

Les Inventeurs ont ainsi mis au point des compositions, utilisations et méthodes qui permettent notamment de réduire les marquages urinaires répétitifs qui interviennent dans le cas d'un stress et/ou d'un état d'anxiété de mammifères non humains liés par exemple à des circonstances ou des changements particuliers dans leurs environnements immédiats. Également, les compositions, utilisations et méthodes selon la description permettent d'améliorer les conditions de familiarisation des mammifères non humains avec leur nouvel environnement et/ou d'empêcher par exemple les griffures ou griffades ou la destruction du territoire et/ou de diminuer les manifestations sonores (telles que les miaulements, geignements, grognements). Les effets bénéfiques peuvent se mesurer par la réduction des marquages urinaires et des griffades et par la répétition des contacts physiques ou des frottements avec les nouveaux objets ou individus qui les entourent. Plus globalement, les compositions selon la description, de par leur teneur en acides gras, permettent d'améliorer le comportement général des mammifères non humains vis-à-vis de leur environnement et des personnes présentes dans cet environnement, avec notamment une réduction du stress, de l'anxiété, du marquage urinaire, et/ou des manifestations sonores, ainsi qu'un comportement moins agressif, plus détendu et plus affectueux notamment avec leurs maîtres.

Les Inventeurs ont en outre trouvé de manière surprenante qu'en ajustant convenablement les concentrations d'acide gras (et en particulier de la fraction F3) et de népétalactone, il était possible de diffuser des mélanges de népétalactone et d'acide gras, en particulier de fraction F3 synthétique de phéromone faciale du chat, pendant des périodes supérieures au besoin d'exposition et de traitement du chat.

L'objet de la présente invention est défini par les revendications 1 à 16.

L'invention concerne une composition apaisante pour mammifères non humain comprenant entre 1 et 50% en poids/poids d'au moins un acide gras comprenant entre 5 et 22 atomes de carbone et entre 0,01 et 5% en poids/poids de népétalactone, caractérisée en ce que le(s)dit(s) acide(s) gras et ladite népétalactone sont en solution dans un solvant choisi parmi les alcools, purs ou en mélange avec de l'eau, les paraffines aliphatiques, les glycols éthers ou les polyglycols éther ou un mélange d'au moins deux d'entre eux.

La présente description se rapporte ainsi à une composition apaisante pour mammifères non humains comprenant entre 1 et 50% en poids/poids d'au moins un acide gras comprenant entre 5 et 22 atomes de carbone et entre 0,01 et 5% en poids/poids de népétalactone, caractérisée en ce que le(s)dit(s) acide(s) gras et ladite népétalactone sont en solution dans un solvant.

Dans un mode de réalisation particulier de l'invention, la composition apaisante pour mammifères non humain telle que décrite précédemment est caractérisée en ce que le mammifère non humain est un félin et dans un mode de réalisation plus particulier de l'invention, le félin est un chat domestique.

La composition selon la description est particulièrement efficace sur les chats de manière large, incluant tous les membres de la famille des félidés ou des félins, incluant les chats domestiques, et plus généralement toutes les races de chats, ainsi que le tigre, le lion, le léopard, le lion de montagne, le lynx, le lynx roux, l'ocelot et similaires.

Par acide gras, on entend, selon la description, des acides monocarboxyliques à chaîne hydrocarbonée, saturés ou insaturés, linéaires ou ramifiés. Plus précisément, ces acides gras sont des substances chimiques susceptibles de modifier le comportement ou les réponses physiologiques de l'animal.

Dans un mode de réalisation particulier de l'invention, la composition apaisante pour un félin telle que décrite précédemment est caractérisée en ce que la teneur en acide gras est comprise entre 1% et 20% en poids/poids et de manière encore préférentielle entre 1% et 15% en poids/poids.

De préférence, les acides gras utilisés, seuls ou en mélange, contiennent entre 5 et 22 atomes de carbone ; des exemples non limitatifs en sont les acides caprique, laurique, azélaïque, myristique, palmitique, palmitoléique, oléique, linoléique, linolénique, stéarique, arachidonique, caproïque, pivalique, gammalinoléique, pimélique, eicosapentanoïque, docosahexanoïque, pentadécanoïque, et tridécanoïque.

Dans un mode de réalisation préféré de l'invention, la composition selon l'invention est caractérisée en ce que les acides gras sont choisis parmi l'acide oléique, linoléique, linolénique, palmitique, myristique, azélaïque, pimélique, caprique, laurique ou encore un mélange d'au moins deux acides gras de la liste qui précède. Encore plus préférentiellement, la composition selon la description comprend au moins un acide gras choisi parmi l'acide oléique, pimélique, azélaïque et palmitique.

Les compositions selon la description peuvent contenir n'importe quels dérivés d'acides gras, comme par exemple des esters d'acides gras.

De préférence, les dérivés d'acides gras utilisés, seuls ou en mélange, sont dérivés d'acides gras constitués de 5 à 22 atomes de carbone. Des exemples non limitatifs en sont l'éthyl pimélate, le diethylpimélate, le monomethylnonanedioate, l'éthyl decanoate, l'éthyl laurate, l'ethyl palmitate et l'ethyl oleate.

Selon un mode de réalisation particulier de l'invention, la composition telle que décrite précédemment est caractérisée en ce que les acides gras sont choisis parmi les acides gras constitutifs de la Fraction F3 de phéromones faciales du chat; la composition en acides gras de cette fraction est connue de l'homme du métier car largement décrite dans l'état de la technique ; ce mélange peut par exemple être reconstitué de façon synthétique tel que décrit dans l'exemple 3 qui suit.

Par phéromones, on entend, selon la description, une substance libérée par le corps de certaines espèces agissant comme des messagers entre les individus, ladite substance pouvant servir notamment à communiquer, ou encore comme un attractant spécifique, par exemple dans l'attraction sexuelle.

Une autre des caractéristiques de la composition selon la description est telle que les ingrédients qu'elle comprend sont en solution.

Par solution, on entend que le mélange est liquide et homogène et qu'il ne contient pas de particules solides en suspensions, tous les éléments solides du mélange étant totalement dissouts dans un ou plusieurs solvants miscibles entre eux.

N'importe quel solvant ou mélange de solvants des constituants de la composition peut être envisagé pour diluer la formulation, tant qu'il reste compatible avec les exigences réglementaires liées à la commercialisation et à l'utilisation de produits utilisés dans l'environnement des animaux et de l'homme.

De manière préférentielle, les solvants utilisés pour diluer la formulation sont choisis parmi les alcools, purs ou en mélange avec de l'eau, les paraffines aliphatiques, les glycols éthers ou les polyglycols éther ou un mélange d'au moins deux d'entre eux.

Lorsque le solvant est un alcool mélangé avec de l'eau, ledit mélange comprend une quantité d'eau inférieure ou égale à 30% en poids, de préférence inférieure ou égale à 20% en poids, encore préférentiellement inférieure ou égale à 10% en poids. A titre d'exemple, le mélange eau/alcool est de 30/70, de 20/80 ou encore de 10/90.

Dans un mode de réalisation préféré de l'invention, la composition apaisante telle que décrite précédemment est caractérisée en ce que les paraffines aliphatiques, les glycols éther et les polyglycols éther ont un point d'ébullition compris entre 200°C et 330°C, de préférence entre 230°C et 310°C.

En particulier, les alcools sont choisis parmi les alcools constitués de 1 à 6 atomes de carbone. Des exemples non limitatifs en sont l'éthanol, l'isopropanol, le méthanol, le butanol, l'isobutanol ou le pentanol. De manière préférentielle, l'éthanol et l'isopropanol sont utilisés.

De préférence, les paraffines aliphatiques sont choisies parmi les produits Isopar™ fabriqués par ExxonMobil Chemical Hydrocarbons.

Les glycols éthers sont préférentiellement choisis parmi les mono glycols éther de méthyle, de propyle ou de butyle.

Dans un mode de réalisation préféré de l'invention, la composition apaisante telle que décrite précédemment est caractérisée en ce que les polyglycols éther sont choisis parmi les mono-, di-, ou tri- polypropylènes glycol éther de méthyle, d'éthyle, de propyle ou de butyle.

Dans un mode de réalisation de l'invention, la composition apaisante telle que décrite précédemment est caractérisée en ce que la népétalactone utilisée est d'origine synthétique ou introduite sous forme d'extrait de plantes.

Selon un mode de réalisation particulier de l'invention, , la composition apaisante telle que décrite précédemment est caractérisée en ce que la népétalactone utilisée est issue d'un extrait de *Nepeta cataria.*

La composition selon la description peut aussi comprendre optionnellement des constituants additionnels tels que, par exemple, des antioxydants, des stabilisants, des promoteurs de séchages, des colorants, des parfums. De tels constituants ne devront bien entendu pas avoir d'effets antagonistes sur les constituants attractifs et apaisants de la composition. De préférence, la composition selon la description est exempte de cyclodextrine et/ou d'agents antibactériens irritants tels que la chlorhexidine.

Selon un mode de réalisation particulier, la composition apaisante pour mammifères non humains consiste entre 1 et 50% en poids/poids d'au moins un acide gras comprenant entre 5 et 22 atomes de carbone et entre 0,01 et 5% en poids/poids de népétalactone et, optionnellement, des constituants additionnels tels que, par exemple, des antioxydants, des stabilisants, des promoteurs de séchages, des colorants, des parfums, caractérisée en ce que le(s)dit(s) acide(s) gras et ladite népétalactone sont en solution dans un solvant ; ces ingrédients et leurs concentrations préférées étant tels que définis précédemment.

La composition selon la description présente l'avantage d'être stable et homogène, sous la forme d'une solution.

La demanderesse entend par stabilité pendant la durée de l'exposition ou du traitement, qui typiquement est de 30 jours, que la composition conservera au moins 50% de sa teneur initiale en népétalactone (ce qui correspond à un temps de demi-vie de la népétalactone supérieure ou égal à la durée du traitement).

Les compositions selon la présente description permettent de prévenir et d'apaiser les chats vis-à-vis du stress ressenti, et en conséquence permettent entre autre d'éviter les futurs marquages, tels que les marquages urinaires ou les griffades.

Par marquage urinaire, on entend, selon la description, un jet d'urine odorant projeté horizontalement sur un support vertical par un mammifère non humain. Le jet d'urine odorant contient des phéromones qui ont notamment un rôle d'alarme pour les congénères.

Par stress, on entend, selon la description, que le mammifère non humain a une appréhension du danger et la crainte accompagnée par la tension consciente, qui se traduit par une forte probabilité de donner des réponses comportementales et émotionnelles de peur. En termes neurobiologiques, cet état de stress est accompagné d'une hyperactivité des systèmes noradrénergiques et de sérotonine.

La présente description se rapporte également à une méthode pour réduire les manifestations du stress ou de l'anxiété chez un mammifère non humain consistant à exposer le mammifère non humain à une composition selon la description ; à une méthode pour prévenir et réduire les marquages urinaires et les griffades chez les chats consistant à exposer le chat à une composition selon la description et encore à une méthode pour faciliter l'adaptation et la socialisation des chatons ou pour augmenter leurs interactions sociales consistant à exposer les chatons à une composition selon la description.

La présente invention se rapporte également à une composition selon l'invention pour son utilisation pour réduire les manifestations du stress ou de l'anxiété chez un mammifère non humain, en particulier les félins, notamment le chat domestique.

Elle se rapporte aussi à une composition selon l'invention pour son utilisation pour prévenir et réduire les marquages urinaires et les griffades chez les chats et pour faciliter l'adaptation et la socialisation des chatons ou pour augmenter leurs interactions sociales.

De manière préférentielle, la composition selon la description est mise en contact avec l'animal par diffusion dans la zone dans laquelle l'animal doit évoluer, par le biais d'un vaporisateur ou d'un diffuseur électrique ou de tout autre système connu de l'Homme du Métier qui permet à la composition de diffuser dans l'environnement du mammifère non humain.

Ainsi, la présente invention se rapporte à l'utilisation d'une composition selon l'invention dans des diffuseurs électriques ou des vaporisateurs pour diffuser une composition apaisante et à l'utilisation d'une composition selon l'invention dans des diffuseurs électriques ou des vaporisateurs pour diffuser une composition pour prévenir les marquages tels que les marquages urinaires et les griffades chez les chats.

Par diffusion on entend, selon la description, la propagation du produit par relargage que cela soit par vaporisation ou par le biais d'un diffuseur électrique.

Par "effet attractant" d'un composé on entend, selon la description, que les chats ou les mammifères non humains sont attirés par l'odeur de ce composé.

De préférence, le procédé de fabrication de la composition selon l'invention consiste à mettre le ou les acide(s) gras en solution dans le solvant à chaud puis, lorsque le mélange est homogène, introduire à température ambiante dans ledit mélange la népétalactone d'origine synthétique ou sous forme d'extrait de plante jusqu'à l'obtention d'une composition homogène. Ce procédé de fabrication se fera préférentiellement sous agitation constante, dans un réacteur fermé. De manière préférentielle, le mélange d'acide gras à chaud se fera entre 40°C à 80°C. De manière encore plus préférentielle, ce mélange peut se faire à 45°C.

### Figures

La **Figure 1** est un graphique comprenant les courbes illustrant la dégradation au cours du temps (en jours) de la népétalactone à 40°C pour les exemples 4 à 7.
La **Figure 2** est un histogramme représentant le pourcentage de réduction des scores comportementaux évalués lors d'un stress par des chats exposés à la composition préparée selon l'exemple 5, normalisé sur le contrôle positif, tel que décrit dans l'exemple 9.

### Exemples comparatifs:

Le suivi de stabilité de népétalactone est effectué par Chromatographie en Phase Gazeuse (CPG). Les échantillons sont gardés en étuve ventilée à 40°C et des prélèvements sont analysés régulièrement. Le produit est considéré comme suffisamment stable lorsque le temps de demi-vie de la népétalactone dans le mélange à 40°C est supérieur à 20 jours (ce qui revient à 30 jours à 25°C).

Les résultats sont exprimés en pourcentage massique de népétalactone par rapport à la quantité initiale. Les analyses ont été effectuées dans les conditions suivantes : appareil de CPG de marque Hewlett Packard (5890 Serie II), équipé d'un détecteur FID (Flame Ionisation Detector) et d'une colonne HP5 (Agilent J&W) 30 m x 0,53mm Film 0,88 µm, avec une pression d'hélium de 11 psi, une température d'injecteur de 250°C, une température de détecteur de 280°C. Ont aussi été utilisés : une température de four initiale de 100°C, un temps initial de 3 min; une rampe 1 de 30°C/min, une température finale 1 de 170°C et un temps de palier 1 de 3 min; une rampe 2 de 5°C/min, une température finale 2 de 250°C ; une rampe 3 de 20°C/min, une température finale de 280°C et un temps de palier final de 4 min.

Les échantillons à analyser ont été préparés en diluant 600 mg d'échantillon en stabilité dans de l'éthanol (QSP 20 ml). Le volume d'échantillon injecté dans la CPG est de 1 µl.

La népétalactone utilisée pour l'illustration de l'invention est obtenue dans le commerce auprès de Berjé sous forme d'extrait de *Nepeta cataria* qui contient environ 80% de népétalactone.

### Exemple 1 : Etude la stabilité de la népétalactone à 0,8% en poids dans l'acide linoléique.

A 9,9 g d'acide linoléique est additionné 100 mg d'extrait de *Nepeta cataria.* Cette solution est mise dans une étuve à 40°C. Des prélèvements ont été effectués à 14,5h, 24h et 45h.

L'étude de stabilité à 40°C d'une solution à 0,8% de népétalactone dans l'acide linoléique est présentée dans le tableau I ci-dessous.

**Tableau I : stabilité à 40°C d'une solution 0,8% en poids de népétalactone dans l'acide linoléique**

| ***Temps (jours)*** | **% de dégradation de la népétalactone** |
|---|---|
| *0* | --- |
| *0.5* | 4,4% |
| *1* | 15% |
| *2* | 19,5% |

Cet exemple montre qu'à un taux de 0,8% en poids dans l'acide linoléique, après seulement 1 jour la composition a perdu 15% de sa teneur en népétalactone ce qui rend inexploitable une telle composition puisque l'effet de la népétalactone diminuerait trop rapidement pendant la durée d'utilisation de la composition en tant qu'apaisant de mammifère non humain. Le temps de demi-vie de cette composition est estimé à 12 jours seulement.

### Exemple 2 : Etude de la stabilité de la népétalactone dans un mélange 85/15 Acide linoléique/ Extrait de Nepeta cataria.

A 8,5 g d'acide linoléique est additionné 1,5 g d'extraits de *Nepeta cataria.* Cette solution est mise dans une étuve à 40°C. Des prélèvements ont été effectués régulièrement pour analyse.

L'étude de stabilité à 40°C d'une solution à 12% en poids par rapport au poids total de la solution de népétalactone dans l'acide linoléique est présentée dans le tableau II ci-dessous.

**Tableau II : stabilité à 40°C d'une solution de 12% de népétalactone dans l'acide linoléique**

| ***Temps en jours*** | **% de dégradation de la népétalactone** |
|---|---|
| *0* | --- |
| *0.5* | 4,4% |
| *1* | 7,5% |
| *2* | 11,7% |

Approximativement 12% de népétalactone est dégradée après 2 jours. Ceci induit un temps de demi-vie de 13 jours ce qui rend impropre ce mélange à l'application souhaitée. Par ailleurs la cinétique plus lente que dans l'exemple 1 suggère que la dégradation de la népétalactone est gouvernée par la teneur en acide gras.

### Exemple 3 : Etude de la stabilité de la népétalactone en présence d'une fraction F3 de phéromone faciale du chat synthétique.

### a) Reconstitution synthétique d'une fraction F3 de phéromone faciale du chat

La Fraction F3 de phéromone faciale du chat reconstituée correspond à celle qui est utilisée dans le produit commercialisé par la société CEVA sous la marque Feliway®. Pour plus de précision, les auteurs ont analysé par GCMS des produits commercialisés sous la marque Feliway® et contenant de la fraction F3 synthétique. La composition identifiée dans les échantillons et reproduite pour les essais est la suivante :

| Composé | Composition de la Fraction F3 selon analyse du produit commercial Feliway® (g) | Quantité des composants utilisés pour fabriquer 100 g de Fraction F3 de phéromone faciale du chat reconstituée (g) |
|---|---|---|
| Pimelic acid | 3,04 | 3,05 |
| Ethyl pimelate | 2,88 | 2,89 |
| Diethylpimelate | 0,42 | 0,42 |
| Azelaic acid | 3,4 | 3,4 |
| Monomethylnonanedioate | 2,7 | 2,7 |
| Decanoic acid | 0,15 | 0,16 |
| Ethyl decanoate | 0,12 | 0,12 |
| lauric acid | 2,9 | 2,89 |
| Ethyl laurate | 0,8 | 0,81 |
| Palmitic acid | 10,85 | 10,86 |
| Ethyl palmitate | 4,6 | 4,59 |
| Cis-13-octadecenoic acid | 40 | 40 |
| Ethyl oleate | 27 | 27 |

La formulation se fait à 45°C en commençant par introduire les dérivés oléiques de manière à bien solubiliser l'ensemble des composants. Stabilité de la Népétalactone dans la fraction F3

### b) Stabilité de la Népétalactone dans la fraction F3

A 9,9 g de la fraction F3 ainsi obtenue à 70% dans l'Ethanol est additionné 100 mg de népétalactone sous forme d'extraits de *Nepeta cataria.* Cette solution est mise dans une étuve à 40°C. Des prélèvements ont été effectués à différents intervalles de temps. L'étude de stabilité à 40°C d'une solution 70% de fraction F3 contenant 0,8% en poids par rapport au poids total de la solution de népétalactone est présentée dans le tableau III ci-dessous.

**Tableau III : stabilité à 40°C d'une solution 0,8% en poids de népétalactone dans une fraction F3 obtenue à 70% dans l'Ethanol**

| ***Temps en jours*** | **% de dégradation de la népétalactone** |
|---|---|
| *0* | --- |
| *1* | 7,4% |
| *2* | 19,7% |

Une dégradation similaire à celle observée dans l'exemple 1 est observée, elle rend là encore inexploitable une telle composition car la teneur en népétalactone diminue trop vite. La cinétique plus rapide que dans l'essai 2 montre que la fraction F3 dégrade plus rapidement la népétalactone que l'acide linoléique.

### Exemple 4a-c : Préparation de solutions alcooliques d'acide linoléique et d'extrait de Nepeta cataria dans un ratio 95/5.

Procédure générale : on dissout 9,5 g d'acide linoléique dans un volume d'isopropanol à 40°C puis après retour à température ambiante, on ajoute 0,5 g de népétalactone sous forme d'extrait de *Nepeta cataria.*

Les solutions préparées sont caractérisées par le volume d'isopropanol utilisé et résumées dans le tableau IV suivant :

**Tableau IV : solutions alcooliques 4a, 4b et 4c d'acide linoléique et d'extrait de Nepeta cataria dans un ratio 95/5**

| | Acide linoleique (g) (%) | Extrait de *Nepeta cataria* (g) (% de népétalactone) | Isopropanol (g) (%) |
|---|---|---|---|
| Exemple 4a | 9.5 (47.5%) | 0.5(2%) | 10 (50%) |
| Exemple 4b | 9.5 (31.7%) | 0.5 (1.36%) | 20 (66%) |
| Exemple 4c | 9.5 (19%) | 0.5 (0.8%) | 40 (80%) |

### Exemple 5 : Préparation de solutions alcooliques de fraction F3 et de népétalactone selon l'invention

On procède comme dans l'exemple 3 en mélangeant 10 g de fraction F3 à 90 g de mélange eau-éthanol dénaturé (10/90) puis on ajoute 0,5 g d'extrait de *Nepeta cataria.* Ces mélanges sont effectués à température ambiante et le mélange final est homogène. La teneur initiale de népétalactone mesurée (par rapport d'air chromatographique et en comparaison avec un étalon pur de népétalactone) est de 0,4% en poids par rapport au poids total de la solution

### Exemple 6 : Préparation de solutions paraffiniques de fraction F3 et de népétalactone.

On prépare la fraction F3 comme dans l'exemple 3 en mélangeant 2 g de fraction F3 dans 97,5 g de paraffine à 45°C. On laisse revenir à température ambiante puis on rajoute 0,5 g de népétalactone. Après une brève agitation on obtient un mélange translucide. Le dosage de la népétalactone dans le mélange est fait par rapport à un étalon externe.

### Exemple 7 : Préparation des solutions polyglycolique de fraction F3 et de népétalactone.

On prépare la fraction F3 comme dans l'exemple 5 en mélangeant 2 g de fraction F3 dans 97,5 g de propyl-dipropylène glycol ether à 35°C. On laisse revenir à température ambiante puis on rajoute 0,5 g de népétalactone. Après une brève agitation on obtient un mélange translucide.

### Exemple 8 : Etude de la stabilité des compositions des exemples 4 à 7

Tous les échantillons sont conditionnés en enceintes climatiques à 40°C à 75% d'humidité, tête en bas pour les sprays (pour garantir l'étanchéité des flacons et éviter des réactions secondaires que pourrait entrainer la présence d'air).

Les résultats de l'étude de stabilité sont présentés dans le tableau V ci-dessous :

**Tableau V : Evolution de la teneur en Népétalactone dans le temps par rapport à la teneur initiale (en % résiduel)**

| nombre de jours | 0 | 1 | 2 | 7 | 45 | 90 |
|---|---|---|---|---|---|---|
| Exemple 4a | 100 | 98 | 95 | 83 | *40* | *15* |
| Exemple 4b | 100 | 100 | 96 | 87 | *50* | *25* |
| Exemple 4c | 100 | 100 | 97 | 89 | *53* | *30* |
| Exemple 5 | 100 | 100 | 100 | 100 | 96 | 83 |
| Exemple 6 | 100 | 100 | 100 | 100 | 100 | 105 |
| Exemple 7 | 100 | 100 | 100 | 103 | 103 | 100 |

(Les valeurs supérieures à 100 sont dues à la précision de la mesure ; les valeurs en italiques sont extrapolées sur une courbe de tendance avec un coefficient de corrélation supérieur à 99%)

Tous ces exemples ont un temps de demi-vie de la népétalactone dans le mélange à 40°C supérieur à 20 jours. Comme attendu, on voit que la cinétique de dégradation ralentit en fonction de la diminution de la concentration. L'évolution des cinétiques en fonction de la concentration indique que la limite de composition permettant d'avoir un temps de demi-vie à 40°C supérieur à 20 jours est environ de 45% d'isopropanol, 50% d'acide linoléique et 5% d'extrait de népétalactone.

### Exemple 9 : Etude d'efficacité des compositions selon l'invention

L'étude est réalisée avec un groupe de 24 chats adultes sains ayant au moins 12 mois au début de l'étude, divisé en quatre groupes. La mesure de la pression artérielle n'a jamais été effectuée sur aucun des chats : cette manipulation constitue l'évènement générant le stress de l'animal dans cet exemple.

Deux produits ont été testés :
- la composition issue de la présente invention préparée selon l'exemple 5 dans un vaporisateur de 20 ml ;
- le produit commercial Feliway® vaporisateur 20 mL (Ceva, France), qui contient un analogue synthétique de la Fraction F3 (10%), et de l'ethanol q.s.p. 20 ml et qui représente le contrôle positif qui permet de normaliser les résultats obtenus avec la composition préparée selon l'exemple 5.

L'étude comprend une période d'administration de 5 jours et une période de sevrage d'au moins 9 jours. 2 groupes de 6 chats ont reçu le produit testé en premier, et les 2 autres le contrôle positif. Tous les chats ont ensuite changé en prenant l'autre produit de sorte que chaque produit a été testé par les 24 chats.

La mesure de la pression artérielle de chaque chat a été effectuée au début et à la fin de la période d'exposition à la composition. Cette dernière a été pulvérisée durant trois jours consécutifs suivant le jour où la première mesure de la pression artérielle a été prise et le jour où la deuxième mesure de la pression artérielle a été réalisée. L'intégralité des produits a été pulvérisée en 5 applications (volume de produit de 0,15 ml par application) dans chaque coin de chaque salle d'étude 3 fois par jour.

L'acceptation de la mesure de la pression artérielle et de la procédure globale a été évaluée grâce à la prise en compte de différentes attitudes somatiques (qui sont caractéristiques des comportements agressifs, méfiants et apaisés) et de l'intensité de ces comportements. Cette évaluation permet l'obtention d'un score comportemental de bien être, qui se traduit par un score faible lorsque le chat est sensible au stress, et un score important lorsque le chat est apaisé.

Ces observations comportementales ont été effectuées à différents temps : 30-40 minutes avant la manipulation, pendant la manipulation, 30 à 40 minutes après la manipulation, et 4-5 heures après la manipulation.

Afin d'évaluer l'impact de la nouvelle composition, la moyenne des scores de stress obtenu avec chacun des produits (produit testé et le contrôle positif Feliway®) a été calculée aux différents temps cités précédemment, lors du jour 1 et de nouveau le jour 5.

La différence de scores entre les jours 1 (avant l'exposition à la composition) et 5 (à la fin de la période d'exposition) a ensuite été comparée pour la composition préparée selon l'exemple 5 normalisée en utilisant le contrôle positif à ces points d'évaluation pour identifier l'impact de la nouvelle composition.

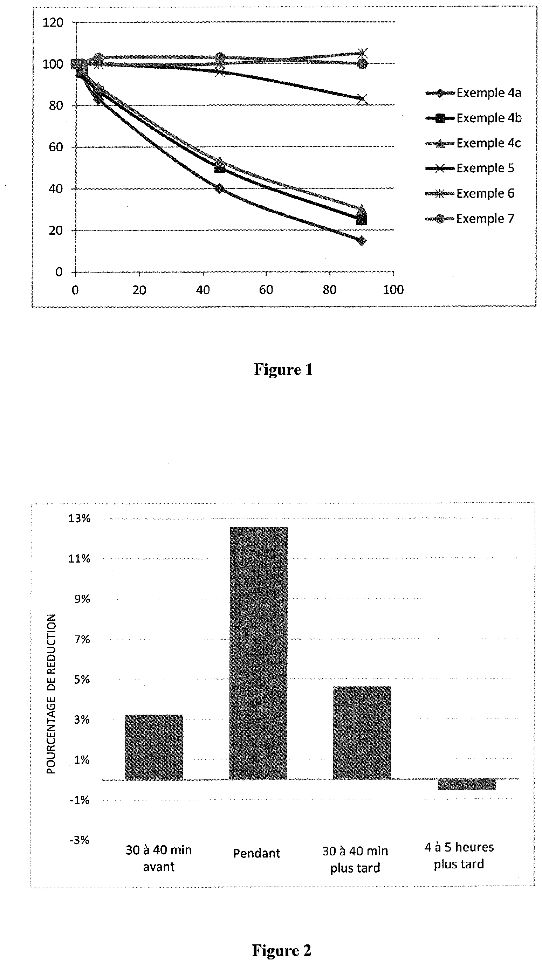

Les résultats de l'étude ont montré une différence entre le contrôle positif et le produit testé selon l'invention à des moments différents (figure 2). Au cours de l'événement stressant central (mesure de la pression artérielle), le produit testé selon l'invention a fourni une réduction statistiquement significative de l'indice de stress par rapport au contrôle positif. 4 à 5 heures après la mesure de la pression artérielle une très faible différence numérique a été notée, sans signification statistique.

L'association de l'analogue synthétique de la fraction F3 et de la népétalactone permet une meilleure gestion du stress chez le chat domestique que chez la fraction F3 seule (Feliway®). Une telle association peut être un nouvel outil pour la gestion de désordres comportementaux modérés, notamment dans le cas du stress immédiat ou aigu. Cette composition peut aussi être utilisée pour faciliter l'adaptation et la socialisation des mammifères non humains, en particuliers les chatons, et augmenter les interactions sociales et le comportement de jeu.

## Revendications

1. Composition apaisante pour mammifères non humain comprenant entre 1% et 50% en poids/poids d'au moins un acide gras comprenant entre 5 et 22 atomes de carbone et entre 0,01% et 5% en poids/poids de népétalactone, **caractérisée en ce que** le(s)dit(s) acide(s) gras et ladite népétalactone sont en solution dans un solvant choisi parmi les alcools, purs ou en mélange avec de l'eau, les paraffines aliphatiques, les glycols éthers, les polyglycols éther ou un mélange d'au moins deux d'entre eux.

2. Composition selon la revendication 1 **caractérisée en ce que** le mammifère non humain est un félin.

3. Composition selon la revendication 2 **caractérisée en ce que** le félin est un chat domestique.

4. Composition selon l'une des revendications 1 à 3, **caractérisée en ce que** la teneur en acide gras est comprise entre 1% et 20% en poids/poids et de manière encore préférentielle entre 1% et 15% en poids/poids.

5. Composition selon l'une des revendications 1 à 4 **caractérisée en ce que** les acides gras sont choisis parmi l'acide oléique, linoléique, linolénique, palmitique, myristique, azélaique, pimélique, caprique, laurique ou un mélange d'au moins deux d'entre eux.

6. Composition selon l'une des revendications 1 à 5 **caractérisée en ce que** les acides gras sont choisis parmi les acides gras constitutifs de la Fraction F3 de phéromone faciale du chat.

7. Composition selon l'une quelconque des revendications précédentes, **caractérisée en ce que** les paraffines aliphatiques, les glycols éther et les polyglycols éther ont un point d'ébullition compris entre 200°C et 330°C, de préférence entre 230°C et 310°C.

8. Composition selon l'une quelconque des revendications précédentes, **caractérisée en ce que** les polyglycols éther sont choisis parmi les mono-, di-, ou tri- polypropylènes glycol éther de méthyle, d'éthyle, de propyle ou de butyle.

9. Composition selon l'une des revendications 1 à 8 pour laquelle la népétalactone utilisée est d'origine synthétique ou introduite sous forme d'extrait de plantes.

10. Composition selon la revendication 9 **caractérisée en ce que** la népétalactone utilisée est issue d'un extrait de *Nepeta cataria.*

11. Utilisation d'une composition selon l'une des revendications 1 à 10 dans des diffuseurs électriques ou des vaporisateurs pour diffuser une composition apaisante.

12. Utilisation d'une composition selon l'une des revendications 1 à 10 dans des diffuseurs électriques ou des vaporisateurs pour diffuser une composition pour prévenir les marquages tels que les marquages urinaires et les griffades chez les chats.

13. Composition selon l'une des revendications 1 à 10 pour son utilisation pour réduire les manifestations du stress ou de l'anxiété chez un mammifère non humain.

14. Composition selon l'une des revendications 1 à 10 pour son utilisation pour prévenir et réduire les marquages urinaires et les griffades chez les chats.

15. Composition selon l'une des revendications 1 à 10 pour son utilisation pour faciliter l'adaptation et la socialisation des chatons ou pour augmenter leurs interactions sociales.

16. Procédé de fabrication d'une composition selon l'une des revendications 1 à 10 consistant à mettre le ou les acide(s) gras en solution dans le solvant à chaud puis, lorsque le mélange est homogène, introduire à température ambiante dans ledit mélange la népétalactone d'origine synthétique ou sous forme d'extrait de plante jusqu'à l'obtention d'une composition homogène.

## Patentansprüche

1. Besänftigende Zusammensetzung für nicht-humane Säugetiere, umfassend zwischen 1 % und 50 % Gewicht/Gewicht mindestens eine Fettsäure, umfassend 5 bis 22 Kohlenstoffatome, und zwischen 0,01 % und 5 % Gewicht/Gewicht Nepetalacton, **dadurch gekennzeichnet, dass** die Fettsäure(n) und das Nepetalacton in Lösung in einem Lösungsmittel vorliegen, ausgewählt aus Alkoholen, rein oder im Gemisch mit Wasser, aliphatischen Paraffinen, Glykolethern, Polyglykolethern oder einem Gemisch aus mindestens zwei davon.

2. Zusammensetzung nach Anspruch 1, **dadurch gekennzeichnet, dass** das nicht-humane Säugetier eine Katze ist.

3. Zusammensetzung nach Anspruch 2, **dadurch gekennzeichnet, dass** die Katze eine Hauskatze ist.

4. Zusammensetzung nach einem der Ansprüche 1 bis 3, **dadurch gekennzeichnet, dass** der Fettsäuregehalt zwischen 1 % und 20 % Gewicht/Gewicht und noch bevorzugter zwischen 1% und 15% Gewicht/Gewicht liegt.

5. Zusammensetzung nach einem der Ansprüche 1 bis 4, **dadurch gekennzeichnet, dass** die Fettsäuren ausgewählt sind aus ÖI-, Linol-, Linolen-, Palmitin-, Myristin-, Azelain-, Pimelin-, Caprin-, Laurinsäure oder einem Gemisch aus mindestens zwei davon.

6. Zusammensetzung nach einem der Ansprüche 1 bis 5, **dadurch gekennzeichnet, dass** die Fettsäuren ausgewählt sind aus den Fettsäuren, die die F3-Fraktion des Gesichtspheromons der Katze bilden.

7. Zusammensetzung nach einem der vorstehenden Ansprüche, **dadurch gekennzeichnet, dass** die aliphatischen Paraffine, Glykolether und Polyglykolether einen Siedepunkt im Bereich zwischen 200 °C und 330 °C, vorzugsweise zwischen 230 °C und 310 °C, aufweisen.

8. Zusammensetzung nach einem der vorstehenden Ansprüche, **dadurch gekennzeichnet, dass** die Polyglykolether ausgewählt sind aus Methyl-, Ethyl-, Propyl- oder Butyl- Mono-, Di- oder Tripolypropylenglykolethern.

9. Zusammensetzung nach einem der Ansprüche 1 bis 8, für die das verwendete Nepetalacton synthetischen Ursprungs ist oder als Pflanzenextrakt eingebracht wird.

10. Zusammensetzung nach Anspruch 9, **dadurch gekennzeichnet, dass** das verwendete Nepetalacton aus einem Extrakt von *Nepeta cataria* stammt.

11. Verwendung einer Zusammensetzung nach einem der Ansprüche 1 bis 10 in elektrischen Diffusoren oder Verdampfern zum Diffundieren einer besänftigenden Zusammensetzung.

12. Verwendung einer Zusammensetzung nach einem der Ansprüche 1 bis 10 in elektrischen Diffusoren oder Verdampfern zum Diffundieren einer Zusammensetzung zum Vermeiden von Markierungen wie Urinmarkierungen und Kratzern bei Katzen.

13. Zusammensetzung nach einem der Ansprüche 1 bis 10 zur Verwendung zum Verringern von Stress- oder Angstmanifestationen bei einem nicht-humanen Säugetier.

14. Zusammensetzung nach einem der Ansprüche 1 bis 10 zur Verwendung zum Vermeiden und Verringern von Urinmarkierungen und Kratzern bei Katzen.

15. Zusammensetzung nach einem der Ansprüche 1 bis 10 zur Verwendung zum Erleichtern der Anpassung und Sozialisierung von Kätzchen oder zum Steigern ihrer sozialen Interaktionen.

16. Verfahren zum Herstellen einer Zusammensetzung nach einem der Ansprüche 1 bis 10, das darin besteht, die Fettsäure(n) in dem warmen Lösungsmittel zu lösen, anschließend, wenn das Gemisch homogen ist, bis zum Erhalt einer homogenen Zusammensetzung Nepetalacton synthetischen Ursprungs oder in Form von Pflanzenextrakt bei Umgebungstemperatur in dieses Gemisch einzubringen.

## Claims

1. Soothing composition for non-human mammals comprising between 1% and 50% w/w of at least one fatty acid comprising between 5 and 22 carbon atoms and between 0.01% and 5% w/w of nepetalactone, **characterised in that** said fatty acid or acids and said nepetalactone are in solution in a solvent selected from among alcohols, pure or in a mixture with water, aliphatic paraffins, ether glycols, ether polyglycols or a mixture of at least two of them.

2. Composition according to claim 1, **characterised in that** the non-human mammal is a feline.

3. Composition according to claim 2, **characterised in that** the feline is a domestic cat.

4. Composition according to one of claims 1 to 3, **characterised in that** the fatty acid content is between 1% and 20% w/w and more preferably between 1% and 15% w/w.

5. Composition according to one of claims 1 to 4, **characterised in that** the fatty acids are selected from among oleic, linolenic, linolenic, palmitic, myristic, azelaic, pimelic, capric, lauric acid or a mixture of at least two of them.

6. Composition according to one of claims 1 to 5, **characterised in that** the fatty acids are selected from among the fatty acids that constitute the Fraction F3 of facial pheromone of the cat.

7. Composition according to any one of the preceding claims, **characterised in that** aliphatic paraffins, ether glycols and ether polyglycols have a boiling point comprised between 200°C and 330°C, preferably between 230°C and 310°C.

8. Composition according to any one of the preceding claims, **characterised in that** the ether polyglycols are selected from among mono-, di-, or tri- polypropylene glycol methyl, ethyl, propyl or butyl ether.

9. Composition according to one of claims 1 to 8, for which the nepetalactone used is of synthetic origin or introduced in the form of a plant extract.

10. Composition according to claim 9, **characterised in that** the nepetalactone used originates from a *Nepeta cataria* extract.

11. Use of a composition according to one of claims 1 to 10, in electric diffusers or vaporisers to diffuse a soothing composition.

12. Use of a composition according to one of claims 1 to 10, in electric diffusers or vaporisers to diffuse a composition to prevent sprayings such as urinary spraying and scratching in cats.

13. Composition according to one of claims 1 to 10, for its use to reduce the manifestations of stress or anxiety in a non-human mammal.

14. Composition according to one of claims 1 to 10, for its use to prevent and reduce urinary spraying and scratching in cats.

15. Composition according to one of claims 1 to 10, for its use to facilitate the adaptation and the socialisation of kittens or to increase their social interactions.

16. Method for manufacturing a composition according to one of claims 1 to 10, consisting of putting the fatty acid or acids in solution in a hot solvent then, when the mixture is homogenous, introducing at ambient temperature into said mixture, the nepetalactone of synthetic origin or in the form of a plant extract until a homogenous composition is obtained.
